Europäisches Patentamt

European Patent Office

Office européen des brevets

(11) Veröffentlichungsnummer : **0 104 186**
**B1**

(12) # EUROPÄISCHE PATENTSCHRIFT

(45) Veröffentlichungstag der Patentschrift :
**09.10.85**

(51) Int. Cl.⁴ : **A 61 F   2/00//** A61L17/00,
A61L27/00

(21) Anmeldenummer : **83900906.5**

(22) Anmeldetag : **19.03.83**

(86) Internationale Anmeldenummer :
**PCT/DE 83/00050**

(87) Internationale Veröffentlichungsnummer :
**WO/8303346 (13.10.83 Gazette 83/24)**

(54) VERFAHREN ZUR HERSTELLUNG BIOINAKTIVER TEILBEREICHE VON AUS BIOAKTIVEN MATERIALIEN BESTE-
HENDEN PROTHESEN ODER PROTHESENTEILEN SOWIE DARAUS HERGESTELLTE PROTHESEN.

(30) Priorität : 26.03.82 DE 3211210

(43) Veröffentlichungstag der Anmeldung :
04.04.84 Patentblatt 84/14

(45) Bekanntmachung des Hinweises auf die Patenterteilung : 09.10.85 Patentblatt 85/41

(84) Benannte Vertragsstaaten :
DE FR GB

(56) Entgegenhaltungen :
GB-A- 2 010 122
US-A- 3 919 723

(73) Patentinhaber : ERNST LEITZ WETZLAR GMBH
Ernst-Leitz-Strasse 30 Postfach 20 20
D-6330 Wetzlar 1 (DE)

(72) Erfinder : DEUTSCHER, Klaus
Lerchenweg 20
D-6330 Wetzlar (DE)
Erfinder : BRÖMER, Heinz
Hundsrück 7
D-6330 Wetzlar-Hermannstein (DE)
Erfinder : FRANEK, Henning
Mühlenkopfstrasse 5
D-6333 Braunfels-Tiefenbach (DE)

## Beschreibung

Die Erfindung betrifft ein Verfahren zum Herstellen bioinaktiver (bioinerter) Teilbereiche von an sich aus bioaktiven Materialien bestehenden Prothesen bzw. Prothesenteilen sowie danach hergestellte Prothesen bzw. Prothesenteile.

Die Verträglichkeit vom Implantatmaterialien ist vom Implantationsort, d. h. von der Art des Lagergewebes für das Implantat, abhängig. Implantate für Aderersatz müssen solche Eigenschaften aufweisen, daß keine thrombogenen Wirkungen von ihren Oberflächen ausgehen, weil sonst die Koagulation des Blutes an den betreffenden Oberflächenbereichen einsetzen würde. Bei Implantaten für Weichgewebeersatz sind die plastischen Eigenschaften von besonderer Wichtigkeit. Bei Implantatmaterialien für den Knochenersatz spielt naturgemäß die mechanische Festigkeit des Materials und die Qualität der Bindung zu dem knöchernen Lagergewebe die Hauptrolle.

Beim Einsetzen von Implantaten in den menschlichen Körper wird aber nur selten lediglich eine einzige Gewebsart tangiert. In vielen Fällen grenzt ein Implantat auch an Weichgewebe. Da die Eigenschaft der Bioaktivität durch besondere chemische Zusammensetzung des Implantatmaterials erzielt wird, ist ein solches Material für die Implantation in andere Gewebe nicht besonders vorteilhaft. So kann z. B. beobachtet werden, daß die Implantation einer aus bioaktivem Material bestehenden Prothese stärkere empfindliche Reaktionen im Weichgewebe auslöst als ein bioinertes Material.

Es ist daher Aufgabe der vorliegenden Erfindung, bei Prothesen aus bioaktivem Material diejenigen Oberflächenteilbereiche, an denen die Eigenschaft der Bioaktivität nicht gefordert wird, zu inaktivieren.

Die Aufgabe wird durch die Patentansprüche 1, 2 und 8 gelöst. Weitere Ausgestaltungen sind in den abhängigen Ansprüchen 3 bis 7 und 9 bis 14 beschrieben.

In den Zeichnungen sind reaktionskinetische Austauschvorgänge als Anschauungsmodell sowie Ausführungsbeispiele der vorliegenden Erfindung schematisch dargestellt.

Es zeigen :

Figur 1 einen Teil einer Bioglaskeramik-Prothese, die in vitro einem wässrigen Angriffsmedium ausgesetzt ist ;

Figur 2 den in Fig. 1 gezeigten Teil nach der chemischen Attacke (« Residual-Bioglaskeramik ») ;

Figur 3 ein Zahnwurzelimplantat ohne Suprastruktur.

Figur 4 ein zylindrisches Prothesenteilstück mit subtraktiver Beschichtung.

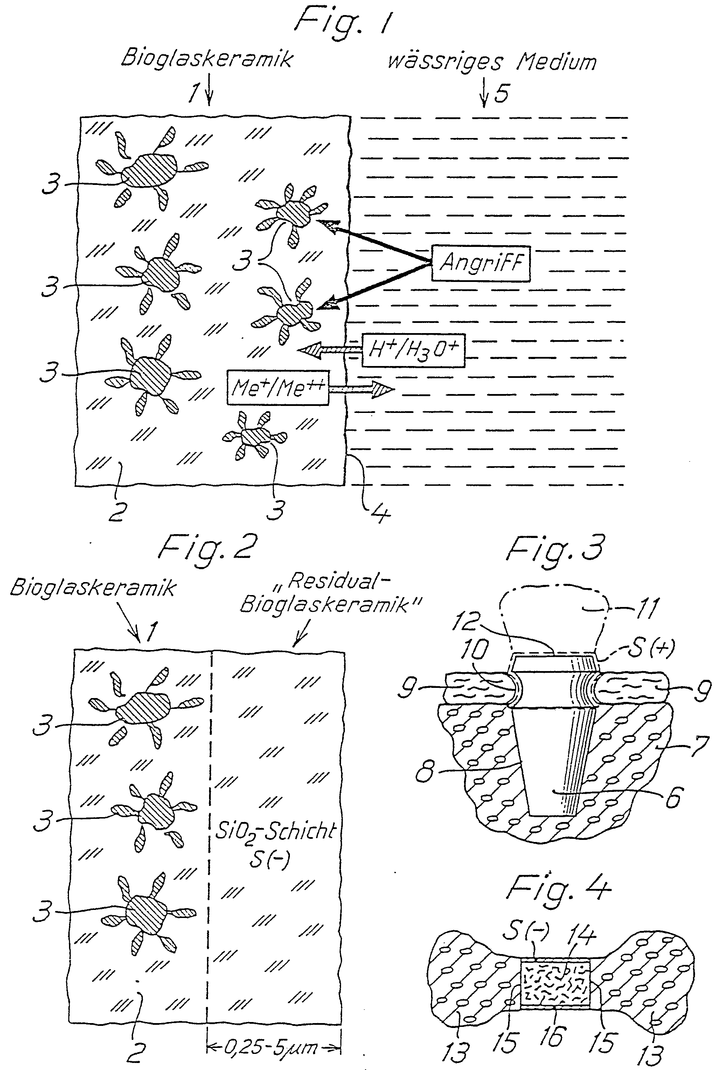

In Fig. 1 ist ein Bioglaskeramik-Material 1 dargestellt, das in einer Glasmatrix 2 eingelagerte, diskrete, kristalline Apatit-Partikel 3 aufweist, die im submikroskopischen Vergrößerungsbereich als eigentümliche, radialstrahlige Aggregationen erkennbar sind. Setzt man die Oberfläche 4 einem wässrigen, sauren Medium 5 aus, erfolgt gezielt ein chemischer Angriff auf die kristalline Apatitphase 3, der dazu führt, daß im Einwirkungsbereich des Mediums 5 der Apatit 3 vollständig aufgelöst wird. Gleichzeitig läuft eine Ionenaustauschreaktion zwischen beweglichen Kationen der Glasmatrix 2 — insbesondere Alkali-($Me^{I-+}$-) und Erdalkali-($Me^{II+}$-)Ionen — und den im Medium 5 vorhandenen $H^+$ und $H_3O^+$-Kationen sowie gegebenenfalls von ein- und zweiwertigen Ionen (Alkalien, Erdalkalien) in Richtung der Bioglaskeramik 1 ab.

Die Geschwindigkeit des chemischen Angriffs auf die Apatitphase 3 soll dabei größer sein als die der Ionenaustauschreaktion, so daß eine apatitfreie, also eine bioinaktivierte und somit « bioinerte » Oberfläche, die wir « Residual-Bioglaskeramik »-Schicht nennen, erzeugt wird.

Die Ionenaustausch- bzw. Angriffsmedien 5 werden dabei zweckmäßigerweise so gewählt, daß im Falle eines eventuell parallel zum Ionenaustausch ablaufenden chemischen Angriffs auf die Glasphase 2 oder Ionenaustausch der reaktionsbestimmende Schritt bleibt, so daß die Austauschreaktion schneller abläuft als der Angriff auf die Glasoberfläche 4.

Für die Reaktionsgeschwindigkeiten und -mechanismen sind bei vorgegebener Bioglaskeramik, wie sie etwa in der DE-C-23 26 100 ausführlich beschrieben ist, die chemischen Verfahrensparameter, wie Art des Mediums, Konzentration, pH-Wert, Zeit und Temperatur, maßgebend.

Beispielsweise läßt sich eine $SiO_2$-Schicht von einer Dicke zwischen 0,25 und 5 μm dadurch im Biokeramik-Oberflächenbereich erzeugen, daß diese zwischen 5 Minuten und 3 Stunden in 0,1 bis 0,001 normaler Salzsäurelösung bei Temperaturen zwischen Raum- und Siedetemperatur behandelt wird.

Daneben lassen sich ähnliche, ebenfalls bioinerte Schichten durch Behandlung in mehreren sauren, wässrigen, organischen oder anorganischen Lösungen, seien diese auch gepuffert oder auch Neutralsalzlösungen, unter Beachtung der genannten Einstellparameter erzeugen.

In Fig. 3 ist als ein Beispiel ein aus bioaktivem Material bestehendes Zahnwurzelimplantat 6 dargestellt, das in seinem mit dem knöchernen Gewebe 7 des Kiefers direkt kontaktierten Oberflächenbereich 8 die qualitativ hochwertige Eigenschaft der « Bioaktivität » zur Geltung kommen läßt. In der für die Gingiva 9 zweckmäßigerweise vorgesehenen Einschnürungszone 10 sowie im weiteren, zur Suprastruktur 11 hinweisenden Oberteil 12 des Implantates wäre hingegen eine Bio-Inaktivierung erwünscht.

Ein anderes Beispiel zeigt — rein schematisch — Fig. 4. Soll ein defektes Skeletteil, im vorliegenden Fall ein Knochen 13, durch ein zylindrisches Prothesen-Zwischenstück 14 « repariert »

werden, ist an den eigentlichen Knochenkontaktflächen 15 — und nur an diesen — ein inniges Verwachsen erwünscht ; an der Mantelfläche 16 hingegen ist die erfindungsgemäße bioinerte Schutzschicht aufgebracht. In Fig. 4 ist sie als subtraktive Schicht S(−) eingezeichnet, während in Fig. 3 die bioinerte Schicht als additive Schutzschicht S(+) dargestellt ist.

Das erfindungsgemäße Verfahren läßt sich — soweit es die Herstellung einer subtraktiven Umwandlungsschicht (Residual-Bioglaskeramik) S(−) betrifft, auf alle Bioglaskeramik-Implantate anwenden und in modifizierter Form auch auf solche aus Bioglas.

Die beschriebenen additiven Beschichtungsverfahren sind außer auf Bioglaskeramik- oder Bioglas-Materialien auch auf alle bekannten an-deren biokompatiblen Implantatmaterialien an-wendbar.

Hinsichtlich der Auswahl der derart behandlungsfähigen Prothesen sind prinzipiell keine Einschränkungen zu machen. So können beispiels-weise Gelenkersatz-, Schädelkalotten- und Na-senscheidewand-Prothesen sowie auch Teilbe-reiche des menschlichen Knochen-, Knorpel-, Bindegewebs- und Körperflüssigkeiten-Systems mit einbezogen werden.

Zusammenstellung der Bezugsziffern und -zeichen

1 — Bioglaskeramik-Material
2 — Glasmatrix der Bioglaskeramik
3 — Apatit-Partikel
4 — Oberfläche von (1)
5 — Wässriges, saures Medium
6 — Zahnwurzelimplantat
7 — knöchernes Gewebe des Kiefers
8 — Kontaktfläche zum knöchernen Gewebe
9 — Gingiva
10 — Einschnürungszone
11 — Suprastruktur
12 — Oberteil
13 — Knochen (Skeletteil)
14 — Prothesen-Zwischenstück
15 — Kontaktfläche zum knöchernen Gewebe
16 — Mantelfläche
S(+) — additive, bioinerte Schutzschicht
S(−) — subtraktive, bioinerte Schutzschicht

**Patentansprüche**

1. Verfahren zur Herstellung bioinaktivierter Oberflächenteilbereiche (10, 12) von aus bioaktiven Materialien bestehenden Prothesen (6), dadurch gekennzeichnet, daß die Teilbereiche (10, 12) dieser Prothesen (6) unter gezielter Stoffzufuhr einer Oberflächen-Nachbehandlung zur Erzeugung mindestens einer additiv aufgebrachten, dauerhaft festsitzenden, in vivo als biochemische Sperrschicht wirkenden, bioinerten Schutzschicht (S(+)) unterworfen werden.

2. Verfahren zur Herstellung bioinaktivierter Oberflächenteilbereiche (16) von aus bioaktiven Materialien bestehenden Prothesen (14), dadurch gekennzeichnet, daß die Teilbereiche (16) dieser Prothese (14) unter gezielter Stoffabfuhr bzw. Stoffaustausch einer chemischen Nachbehandlung zur Erzeugung mindestens einer subtraktiv erhaltenen, permanent festhaftenden, in vivo als biochemische Sperrschicht wirkenden, bioinerten Konversions-(Auslaugungs-) Schicht (S(−)) unterworfen werden.

3. Verfahren nach Anspruch 1, dadurch gekennzeichnet, daß die Schutzschicht (S(+)) durch mindestens einen der folgenden Schritte vorzugsweise aufgebracht wird :

a) Galvanisieren oder Bedampfen ;
b) Aufsputtern ;
c) Abscheiden aus organischen Lösungen oder Aufdampfen im Vakuum ;
d) Tauchen, Sprühen oder Aufstreuen mit nachfolgender thermischer Behandlung ;
e) Eintauchen in Wasserglas mit anschließendem Aufheizen auf ca. 400 °C bzw. Tauchglasieren aus ein- oder mehrkomponentigen Schmelzgemengen ;
f) Simultan-Aufdampfen oder Aufdampfen der Metalle mit nachfolgender Oxidierungsbehandlung.

4. Verfahren nach Anspruch 2, dadurch gekennzeichnet, daß die zu schützenden Prothesenteile mit wässrigen, sauren Lösungen oder mit wässrigen Salzlösungen mit Normalitäten zwischen 0,001 und 0,1 zwischen 5 Minuten und 3 Stunden bei Temperaturen zwischen 20 bis 100 °C behandelt werden.

5. Verfahren nach Anspruch 4, dadurch gekennzeichnet, daß als wässrige, saure Lösung 0,1-0,001 normale Salzsäure (HCl) verwendet wird.

6. Verfahren nach Anspruch 4, dadurch gekennzeichnet, daß als wässrige Salzlösung 0,001 bis 0,25 normale Standard-Azetat-Puffer-Lösung verwendet wird.

7. Verfahren nach Anspruch 2, dadurch gekennzeichnet, daß die beschichteten Teile der Prothese anschließend einer thermischen Versie-gelungs- und/oder Silanisierungsbehandlung un-terworfen werden.

8. Aus bioaktivem Material bestehende, ein-oder mehrstückige Prothese (6, 14) dadurch gekennzeichnet, daß sie in einem oder mehreren Oberflächenteilbereich (en) unlösliches bioinertes Material enthält.

9. Prothese nach Anspruch 8, dadurch gekennzeichnet, daß sie partiell eine Beschichtung aus unlöslichem bioinerten Material aufweist.

10. Prothese nach Anspruch 9, dadurch gekennzeichnet, daß die Beschichtung aus mindestens einer additiv aufgebrachten Schutzschicht (S(+)) mit einer Dicke zwischen 0,25 und 10 μm, vorzugsweise zwischen 1 und 5 μm, besteht.

11. Prothese nach Anspruch 9, dadurch gekennzeichnet, daß die Beschichtung aus mindestens einer subtraktiv erzeugten Konversions-(Auslaugungs)-Schicht (S(−)) mit einer Dicke zwischen 0,25 und 5 μm, vorzugsweise zwischen 1 und 3 μm, besteht.

12. Prothese nach den Ansprüchen 9 bis 11, dadurch gekennzeichnet, daß die Beschichtung aus mindestens einer prothesenkernseitig erzeugten Konversions-(Auslaugungs-)Schicht (S(−)) und mindestens einer auf dieser aufliegenden, additiv aufgebrachten Schutzschicht (S(+)) besteht.

13. Prothese nach mindestens einem der Ansprüche 9, 10 und 12, dadurch gekenzeichnet, daß das bioinerte Material bzw. die bioinerte Schicht aus mindestens einer der folgenden Substanzen besteht :

a) Metalle, wie Gold, Platin, Titan, sowie Metallegierungen ;
b) Kohlenstoff in geeigneten Modifikationen, wie pyrolytischer Kohlenstoff (Graphit) ; Kohlenstoffverbindungen, wie Siliziumkarbid (SiC), Titankarbid (TiC), Borkarbid ($B_4C$) ;
c) Sonderkeramische Werkstoffe, wie hexagonales Bornitrid (BN), Titannitrid (TiN), Siliciumnitrid ($Si_3N_4$) ;
d) teilkristalline anorganische Verbundsysteme, wie Emails ;
e) anorganische Einkomponenten- (z. B. Kieselglas) oder Mehrkomponenten-Gläser ;
f) Oxide, wie Titandioxid ($TiO_2$), Zirkondioxid ($ZrO_2$) und Aluminiumoxid ($Al_2O_3$).

14. Prothese nach mindestens einem der Ansprüche 8, 9, 11 und 12, dadurch gekennzeichnet, daß das bioinerte Material bzw. die bioinerte Schicht aus einer (m) apatitfreien Residual-Bioglaskeramik bzw. -Biokeramik bzw. -Bioglas besteht, welche(s) gegebenenfalls zusätzlich eine Silan-Schicht aufweist.

## Claims

1. Method for the production of biologically inactive partial surface regions (10, 12) of protheses (6) consisting of biologically active materials, characterised thereby, that the partial regions (10, 12) of these protheses (6) are, under targeted supply of substance, subjected to a finishing surface treatment for the production of at least one additively applied, durably adhering, biologically inert protective layer (S(+)) acting in vivo as biochemical barrier layer.

2. Method for the production of biologically inactive partial surface regions (16) of protheses (14) consisting of biologically active material, characterised thereby, that the partial regions (16) of this prothesis (14) are, under targeted supply or exchange of substance, subjected to a chemical finishing treatment for the production of at least one subtractively obtained, permanently adhering, biologically inert conversion (elution)layer (S(−)) acting in vivo as biochemical barrier layer.

3. Method according to claim 1, characterised thereby, that the protective layer (S(+)) is preferably applied by at least one of the following steps :

a) electroplating or vapour-deposition,
b) sputtering,
c) precipitation out of organic solutions/vapour-deposition in vacuum,
d) dipping, spraying or scattering-on with subsequent thermal treatment,
e) immersing in water glass with subsequent heating to about 400 °C or dip-glazing out of single-component or multi-component smelt batches and
f) simultaneous vapour-deposition or vapour-deposition of the metals with subsequent oxidation treatment.

4. Method according to claim 2, characterised thereby, that the prothesis parts to be protected are treated at temperatures of between 20 and 100 °C for 5 minutes to 3 hours with aqueous acid solutions or with aqueous saline solutions at molecular concentrations between 0.001 and 0.1.

5. Method according to claim 4, characterised thereby, that hydrochloric acid (HCl) at a molecular concentration of 0.1 to 0.001 is used as aqueous acid solution.

6. Method according to claim 4, characterised thereby, that standard acetate buffer solution at a molecular concentration of 0.001 to 0.25 is used as aqueous saline solution.

7. Method according to claim 2, characterised thereby, that the coated parts of the prothesis are subsequently subjected to a thermal sealing and/or silanising treatment.

8. One-piece or multi-piece prothesis (6 ; 14) consisting of biologically active material, characterised thereby, that it contains insoluble, biologically inert material in one or more partial surface regions.

9. Prothesis according to claim 8, characterised thereby, that it in part displays a coating of insoluable, biologically inert material.

10. Prothesis according to claim 9, characterised thereby, that the coating consists of at least one additively applied protective layer (S(+)) of a thickness between 0.25 and 10 micrometres, preferably between 1 and 5 micrometres.

11. Prothesis according to claim 9, characterised thereby, that the coating consists of at least one subtractively produced conversion (elution) layer (S(−)) of a thickness between 0.25 and 5 micrometres, preferably between 1 and 3 micrometres.

12. Prothesis according to the claims 9 to 11, characterised thereby, that the coating consists of at least one conversion (elution) layer (S(−)) produced at the core of the prothesis and at least one additively applied protective layer (S(+)) lying on this.

13. Prothesis according to at least one of the claims 9, 10 and 12, characterised thereby, that the biologically inert material or layer consists of at least one of the following substances :

a) metals, such as gold, platinum, titanium as well as metal alloys,

b) carbon in suitable modifications, such as pyrolytic carbon (graphite), carbon compounds such as silicon carbide (SiC), titanium carbide (TiC) and boron carbide ($B_4C$),

c) special ceramic materials such as hexagonal boron nitride (BN), titanium nitride (TiN) and silicon nitride ($Si_3N_4$),

d) partially crystalline inorganic compounds such as enamels,

e) inorganic single-component glasses (for example flint glass) or multi-component glasses and

f) oxides such as titanium dioxide ($TiO_2$), zirconium dioxide ($ZrO_2$) and aluminium oxide ($Al_2O_3$).

14. Prothesis according to at least one of the claims 8, 9, 11 and 12, characterised thereby, that the biologically inert material or layer consists of an apatite-free residual bio-glass-ceramic or bioceramic material or bioglass, which in a given case additionally displays a silane layer.

## Revendications

1. Procédé de fabrication de zones biologiquement inertes de surface (10, 12) de prothèses se composant de matériaux biologiquement actifs caractérisé en ce que les zones (10, 12) de ces prothèses (6) sont soumises, avec apport voulu de matière, à un post-traitement de surface pour la production d'au moins une couche biologiquement inerte de protection (S(+)) produite par addition, fixée de manière durable et servant, in vivo, de couche biochimique de barrage.

2. Procédé de fabrication de zones de surface (16) biologiquement inactivées de prothèses (14) se composant de matériaux biologiquement actifs caractérisé en ce que les zones (16) de ces prothèses (14) sont soumises, avec extraction voulue de matière ou respectivement échange de matière, à un post-traitement chimique pour la production d'au moins une couche de conversion (extraction) (S(−)) obtenue par soustraction, fixée en permanence, servant, in vivo, de couche biochimique de barrage.

3. Procédé selon la revendication 1 caractérisé en ce que la couche de protection (S(+)) est formée avantageusement par au moins l'une des étapes qui suivent :

a) Galvanisation ou métallisation ;
b) Pulvérisation ;
c) Séparation de solutions organiques ou bien métallisation sous vide ;
d) Immersion, aspersion ou saupoudrage avec traitement thermique subséquent ;
e) Plongée dans du verre soluble avec ensuite chauffage à environ 400 °C ou respectivement, vernissage par immersion dans une masse à l'état fondu à un ou plusieurs composants ;
f) Métallisation et traitement d'oxydation simultanés ou bien métallisation des métaux avec traitement subséquent d'oxydation.

4. Procédé selon la revendication 2 caractérisé en ce que les parties de prothèses à protéger sont traitées avec des solutions acides aqueuses ou avec des solutions aqueuses de sel ayant des normalités comprises entre 0,001 et 0,1, entre 5 minutes et 3 heures à des températures comprises entre 20 et 100 °C.

5. Procédé selon la revendication 4 caractérisé en ce qu'en tant que solution acide aqueuse, on utilise de l'acide chlorhydrique normal à 0,1-0,001 (HCl).

6. Procédé selon la revendication 4 caractérisé en ce qu'en tant que solution aqueuse de sel on utilise une solution normalisée de tampons d'acétates à 0,001-0,25 normale.

7. Procédé selon la revendication 2 caractérisé en ce que les parties enduites de la prothèse sont soumises ensuite à un traitement thermique de scellement et/ou de silanisation.

8. Prothèse (6 ; 14) à une ou plusieurs pièces, se composant d'un matériau biologiquement actif caractérisée en ce qu'elle contient, dans une ou plusieurs zones de surface, un matériau biologiquement inerte insoluble.

9. Prothèse selon la revendication 8 caractérisée en ce qu'elle présente partiellement une enduction d'un matériau biologiquement inerte insoluble.

10. Prothèse selon la revendication 9 caractérisée en ce que l'enduction se compose d'au moins une couche de protection formée par addition (S(+)) d'une épaisseur comprise entre 0,25 et 10 μm, avantageusement entre 1 et 5 μm.

11. Prothèse selon la revendication 9 caractérisée en ce que l'enduction se compose d'au moins une couche de conversion (extraction) (S(−)) produite par soustraction, d'une épaisseur comprise entre 0,25 et 5 μm, avantageusement entre 1 et 3 μm.

12. Prothèse selon les revendications 9 à 11, caractérisée en ce que l'enduction se compose d'au moins une couche de conversion (extraction) produite du côté noyau de la prothèse par soustraction (S(−)) et d'au moins une couche produite par addition (S(+)) disposée sur celle-ci.

13. Prothèse selon au moins l'une des revendications 9, 10 ou 12 caractérisée en ce que le matériau biologiquement inerte ou respectivement la couche biologiquement inerte se compose d'au moins l'une des substances qui suivent :

a) Métaux, comme l'or, le platine, le titane ainsi que des alliages de métaux ;
b) Carbone aux modifications appropriées, comme du carbone pyrolytique (graphite) ; des composés de carbone comme du carbure de silicium (SiC), du carbure de titane (TiC), du

carbure de bore (B$_4$C) ;

c) Des matériaux de céramique spéciale comme du nitrure de bore hexagonal (BN), du nitrure de titane (TiN), du nitrure de silicium (Si$_3$N$_4$) ;

d) Des systèmes composés anorganiques partiellement cristallins comme des émaux ;

e) Des verres inorganiques à un composant (comme du quartz fondu) ou à plusieurs composants ;

f) Des oxydes comme du bioxyde de titane (TiO$_2$), du bioxyde de zirconium (ZrO$_2$) et de l'oxyde d'aluminium (Al$_2$O$_3$).

14. Prothèse selon au moins l'une des revendications 8, 9, 11 ou 12 caractérisée en ce que le matériau biologiquement inerte ou respectivement la couche biologiquement inerte se compose de biovitrocéramique ou respectivement biocéramique ou respectivement bioverre résiduel exempt d'apatite qui le cas échéant présente en plus une couche de silane.

Fig. 1

0 104 186

Bioglaskeramik

wässriges Medium

Fig. 2

Bioglaskeramik

„Residual-Bioglaskeramik"

Fig. 3

Fig. 4